# EUROPEAN PATENT APPLICATION

(11) **EP 2 695 874 A1**
(43) Date of publication of application: **12.02.2014**
(21) Application number: 12005722.9
(22) Date of filing: 07.08.2012
(51) Int. Cl.: C07C 45/72, C07C 49/747, A61K 31/122, A61P 29/00, A61P 35/00

(54) **Myrtucommulone analogues**

(71) Applicant: Universität des Saarlandes, 66123 Saarbrücken (DE)
(72) Inventor: Jauch, Johann, 66564 Ottweiler (DE); Müller, Hans, 66125 Saarbrücken (DE); Werz, Oliver, 07749 Jena (DE); Wiechmann, Katja, 07778 Porstendorf (DE)
(74) Representative: Samson & Partner

(57) **Abstract**

The present invention relates to Myrtucommulone analogues of general formula (I) 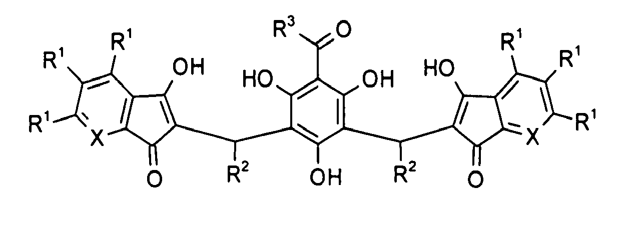 and the preparation thereof.

## Description

### Field of the Invention

The present invention relates to novel Myrtucommulone analogues and the preparation thereof.

### Background of the Invention

Myrtucommulone A and Myrtucommulone B were first isolated in 1974 from the myrtle *Myrtus communis* L., an evergreen flowering shrub which is found in the Mediterranean region of southern Europe and North Africa (Y. Kashman, A. Rotstein, A. Lifshitz, Tetrahedron 1974, 30, 991-997*).* Three years later, they were together with other Myrtucommulones also isolated from other members of the myrtaceae family (M. Lounasmaa, H. -S. Puri, C.-J. Widen, Phytochemistry 1977, 16, 1851-1852).

Myrtucommulone A is of great pharmaceutical interest, since it is highly active against gram-positive bacteria (A. Rotstein, A. Lifshitz, Y. Kashman, Antimicrob. Agents Chemother. 1974, 6, 539-542) and has antioxidative porperties (A. Rosa, M. Deiana, V. Casu, G. Corona, G. Appendino, F. Bianchi, M. Ballero, M. A. Dessi, Free Rad. Res. 2003, 37, 1013-1019). Novel investigations (WO 2008071173 A1, inventors O. Werz, A. Köberle; I. Tretiakova, D. Blaesius, L. Maxia, S. Wesselborg, K. Schulze-Osthoff, J. Cinatl Jr., M. Michaelis, O. Werz, Apoptosis 2008, 13, 119-131) show a pronounced anti-inflammatory acivitiy and a higly selective acitivity against a diversity of tumor cell lines.

WO 2010/022953 (inventor Johann Jauch) discloses an efficient synthesis for Myrtucommulone and certain analogues.

It is desirable to synthesize and investigate further analogues in the search for even more effective compounds in this class of drugs.

### Summary of the Invention

In a first aspect, the present invention relates to Myrtucommulone analogues of general formula (I) 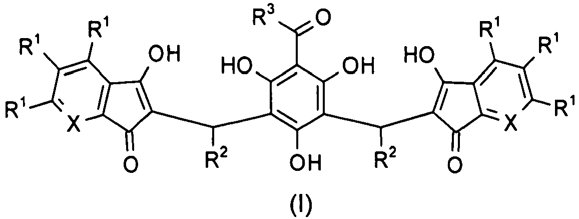 wherein
X is N or CR¹;
each R¹ is independently of each other selected from H and a C₁₋₁₂ linear,
branched or cyclic alkyl, allyl or aryl-C₁₋₄ alkyl group wherein the aryl may be substituted by 1 to 5 substituents independently selected from C₁₋₄ alkyl, C₁₋₄ alkoxy, nitro, halogen (F, Cl, Br und I), cyano, -C(O)-R' and-COOR', wherein R' is C₁₋₄ alkyl, with the proviso that the two indanolone
rings are substituted identically;
each R² is the same substituent and is selected from H and a C₁₋₁₂ linear,
branched or cyclic alkyl, allyl, aryl or aromatic heterocyclic group comprising an aromatic 6-membered ring including one or two nitrogen atoms or an aromatic 5-membered ring including one or two heteroatoms selected from nitrogen and not more than one oxygen atom and not more than one sulfur atom per ring, wherein aryl or the aromatic heterocyclic group may be substituted by one or more substituents independently selected from C₁₋₄ alkyl, C₁₋₄ alkoxy, nitro, halogen (F, Cl, Br und I), cyano, dimethylamino, hydroxyl, -C(O)-R' and -COOR', wherein R' is C₁₋₄
alkyl or H;
R³ is a C₁₋₁₂ linear, branched or cyclic alkyl group, which may be substituted with CO₂H or SO₃H; or an allyl, aryl or aromatic heterocyclic group comprising an aromatic 6-membered ring including one or two nitrogen atoms or an aromatic 5-membered ring including one or two heteroatoms selected from nitrogen and not more than one oxygen atom and not more than one sulfur atom per ring, wherein aryl or the aromatic heterocyclic group may be substituted by one or more substituents independently selected from C₁₋₄ alkyl, C₁₋₄ alkoxy, nitro, halogen (F, Cl, Br und I), cyano, dimethylamino, hydroxyl, -C(O)-R' and -COOR', wherein R' is C₁₋₄ alkyl or H.

In a second aspect the invention relates to a method of preparing of the Myrtucommulone analogue as defined above comprising:
(a) dissolving a compound of formula (IV) 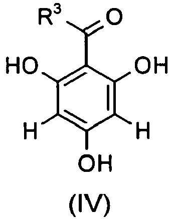 wherein R³ is as defined above, in an aprotic solvent and reacting it with about 1 to about 3 equivalents of a strong base to give a solution or a suspension of a salt of the compound of formula (IV);
(b) dissolving a compound of formula (III) 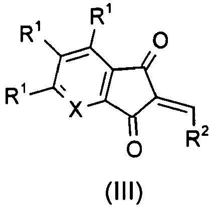 wherein X, R¹ and R² are as defined above, in an aprotic solvent; and
(c) combining the reaction product of (a) with the solution of (b) to obtain a compound of formula (I) 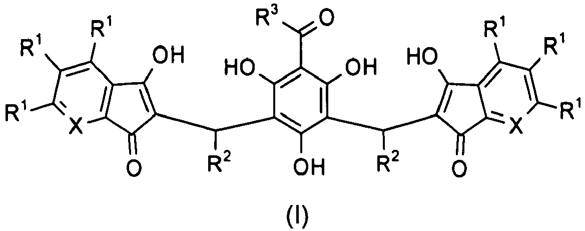 wherein X, R¹, R² and R³ are as defined above.

### Detailed Description

X in formula (I) is nitrogen or CR¹ wherein R¹ is as defined above.

C₁₋₁₂ alkyl in the definitions of R¹, R², and R³ of formula (I) is a linear, branched or cyclic alkyl of 1 to 12 carbon atoms, such as e.g. methyl, ethyl, n-propyl, isopropyl, cyclopropyl, n-butyl, isobutyl, 2-butyl, tert.-butyl, cyclobutyl, n-pentyl, isopentyl, cyclopentyl, hexyl, cyclohexyl, heptyl, cyclohexylmethyl, octyl, cyclohexylethyl, nonyl, decyl, undecyl, dodecyl, and isomers thereof.

Aryl is phenyl or naphthyl.

In aryl C₁₋₄ alkyl, C₁₋₄ alkyl is a linear, branched or cyclic alkyl having 1 to 4 carbon atoms, such as the specific C₁₋₄ alkyl groups listed above.

Aromatic heterocyclic groups comprise an aromatic six-membered ring including one or two nitrogen atoms, such as 2- or 3-pyridyl, pyrimidyl, pyrazyl and pyridazyl, and aromatic five-membered rings including one or two heteroatoms selected from nitrogen, oxygen, and sulfur, wherein no more than one oxygen or sulfur atom is present in each ring. Examples for the latter are thienyl, furanyl, pyrrolyl, imidazolyl, pyrazolyl, thiazolyl, isothiazolyl, oxazolyl, isoxazolyl and 3-indolyl.

Substituents of the aryl or heterocyclic groups are selected from C₁₋₄ alkyl, C₁₋₄ alkoxy, nitro, halogen (F, Cl, Br, and I), cyano, dimethylamino, hydroxyl, -C(O)-R', and -COOR', wherein R' is C₁₋₄ alkyl or hydrogen. The number of substitutents may range from one to the maximum possible number.

Halogen means F, Cl, Br and I.

Cyano is the group CN.

In some embodiments, X is CR¹.

In some embodiments R¹ is independently selected from hydrogen or methyl.

In some embodiments R² is isopropyl.

In some embodiments R³ is selected from isopropyl, butyl, pentyl and hexyl.

The following compounds are particularly preferred:
(Ia) 2,2'-(1,1'-(2,4,6-Trihydroxy-5-isobutyryl-1,3-phenylene)bis(2-methylpropane-1,1-diyl))bis(3-hydroxy-1H-inden-1-one) 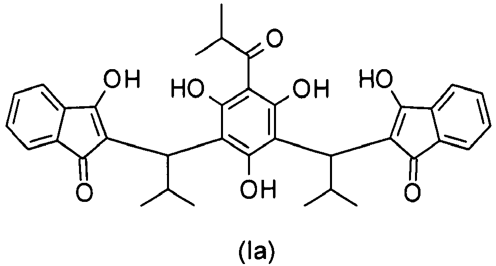
(Ib): 2,2'-(1,1'-(5-Hexanoyl-2,4,6-trihydroxy-1,3-phenylene)bis(2-methylpropane-1,1-diyl))bis(3-hydroxy-1H-inden-1-one) 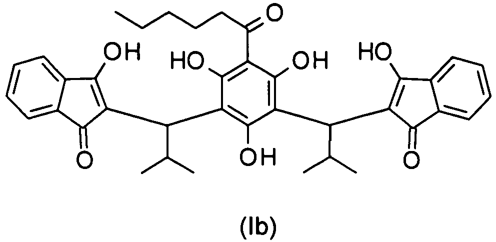
(Ic): 2,2'-(1,1'-(2,4,6-Trihydroxy-5-(3-methylbutanoyl)-1,3-phenylene)bis(2-methylpropane-1,1-diyl))bis(3-hydroxy-1H-inden-1-one) 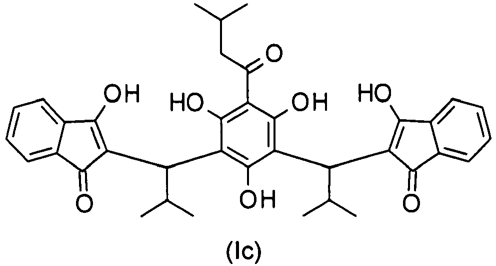
(Id): 2,2'-(1,1'-(5-Benzoyl-2,4,6-trihydroxy-1,3-phenylene)bis(2-methylpropane-1,1-diyl))bis(3-hydroxy-1H-inden-1-one) 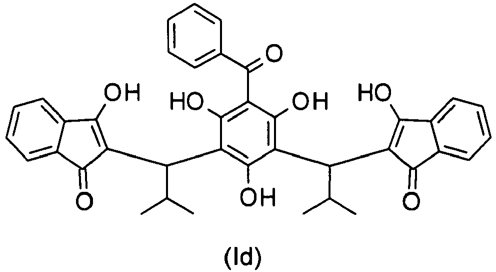
(Ie): 2,2'-(2,4,6-Trihydroxy-5-isobutyryl-1,3-phenylene)bis(phenylmethylene)bis(3-hydroxy-1H-inden-1-one) 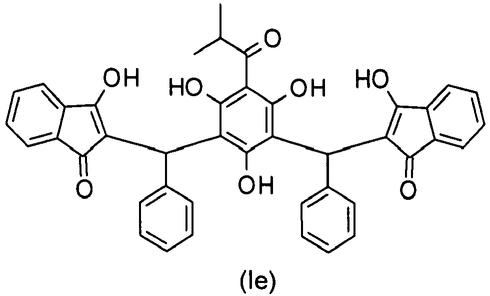
(If): 2,2'-(2,4,6-Trihydroxy-5-(3-methylbutanoyl)-1,3-phenylene)bis(phenylmethylene)bis(3-hydroxy-1H-inden-1-one) 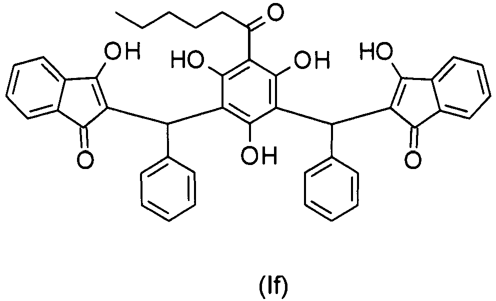
(Ig): 2,2'-(5-Hexanoyl-2,4,6-trihydroxy-1,3-phenylene)bis(phenylmethylene)bis(3-hydroxy-1H-inden-1-one) 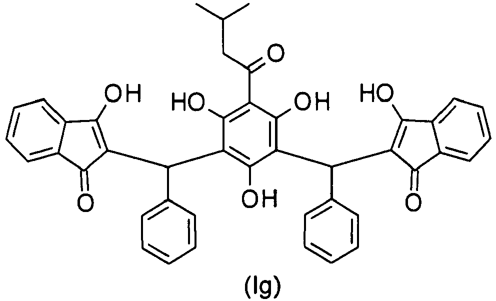
(Ih): 2,2'-(5-Benzoyl-2,4,6-trihydroxy-1,3-phenylene)bis(phenylmethylene)bis(3-hydroxy-1H-inden-1-one)

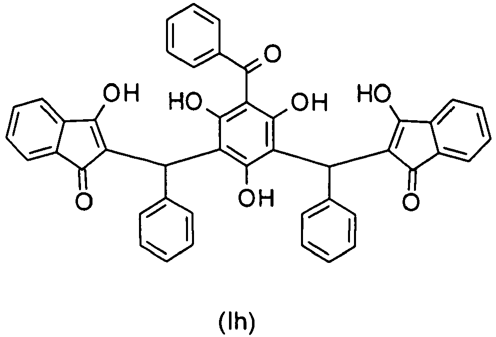

Compounds of formula (III) 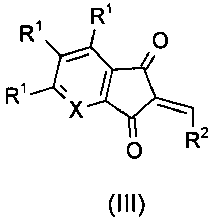 wherein X is N or CR¹ and R¹ and R² are as defined above, are synthesized from compounds of formula (II) 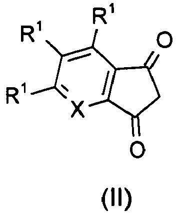 wherein X and R1 are as defined above, and an aldehyde of formula

R²-CHO,

wherein R² is as defined above, according to literature procedures of e.g. Polansky et al. for X = CR¹ (Jörg Bitter, Johannes Leitich, Hans Partale, Oskar E. Polansky, Werner Riemer, Ursula Ritter-Thomas, Brigitte Schlamann und Berthold Stilkerieg, Chem. Ber. 1980, 113, 1020-1032) and e.g. Petrova et al. for X = N (M. Petrova, E. Liepinsh, M. Fleischer, P. Pastors und V. Kampors, Chem. Heterocycl. Comp. 2008, 44, 1024-1032), and literature cited therein, or in an analogous manner.

Compounds of formula (II) are commercially available or can be easily synthesized, see e.g. O. Marvi, M. Giahi, Bull. Korean Chem. Soc. 2009, 30, 2918-2920 and literature cited therein; Raj (S. B.) Rajur, Venugopal N. Rao, Hwa-Ok Kim,Pamela Nagafuji, Xavier Hearult, John D. Williams Norton P. Peet, Synth. Commun. 2009, 39, 626-635, and literature cited therein.

Acylphloroglucinol of formula (IV) 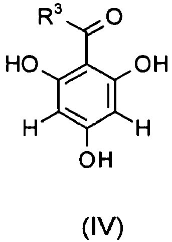 wherein R³ is as defined above, can be prepared as described in Example 5 of WO 2010/022953 or in an analogous manner using Friedel-Crafts acylation.

If C₁₋₁₂ alkyl is substituted with -COOH or -SO₃H, the Friedel-Crafts acylation is, for example, carried out with an acylation reagent, which is synthesized from a diacid by mono esterification and transformation of the free acid into the acid chloride. After acylation of the aromatic core the ester is hydrolyzed.

In step (a) of the synthesis of the compounds of formula (I), an acylphloroglucinol of formula (IV) 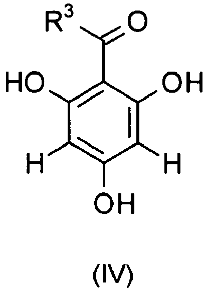 wherein R³ is as defined above, is dissolved in an aprotic solvent and is deprotonated with about 1 to about 3 equivalents of a strong base to obtain a solution or suspension of the corresponding phenoxide salt. The solvent used in step a) is an aprotic solvent, such as acyclic or cyclic ethers (e.g. diethyl ether, dimethoxy ethane, THF or dioxane), dimethylsulfoxide or dimethylformamide. Dimethoxyethane or THF is preferred.

The strong base to deprotonate compound (IV) may be selected from an amide, such as lithium diisopropyl amide, an alkoxide, such as potassium tert-butoxide, or a hydride, such as sodium hydride, potassium hydride or calcium hydride. Sodium hydride is preferred.

Compounds of formula (IV) are deprotonated between about -40 °C and the boiling point of the solvent, preferably at about room temperature. The reaction time for the deprotonation reaction is between about 3 min and about 2 h, preferably about 10 min.

In step (b) of the synthesis of compounds of the formula (I), a compound of formula (III) 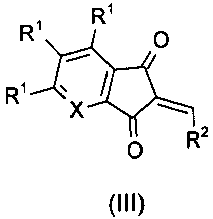 wherein X is N or CR¹ and R¹ and R² are as defined above, is dissolved in an aprotic solvent, and this solution is combined with, preferably added to the solution or suspension obtained in step a), to obtain a compound of formula (I) 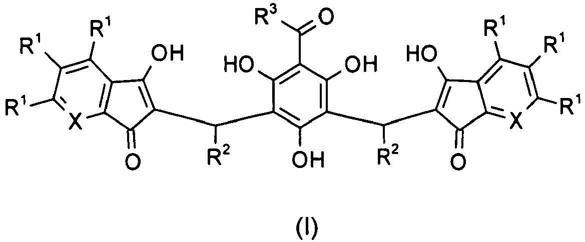 wherein R¹, R² and R³ are as defined above.

The solvent used in step (b) is an aprotic solvent, such as acyclic or cyclic ethers (e.g. diethyl ether, dimethoxy ethane, THF or dioxane), dimethylsulfoxide or dimethylformamide. Dimethoxyethane or THF is preferred.

The reaction of step (b) may generally be carried out for about 10 minutes to about 5 hours, for example at a temperature of about 0 °C to the boiling temperature of the solvent. Preferably, the reaction is carried out for about one hour at about room temperature. Furthermore, the reaction of step (b) is preferably carried out under an inert gas atmosphere.

After the usual processing of the reaction product, e.g. as described in Examples 1-8, a mixture of the meso form and the two enantiomers of the compound of claim 1 is obtained.

The following examples illustrate the invention.

### Examples

### Synthesis Example 1- Synthesis of 2-Isobutylidene-indandione-1,3

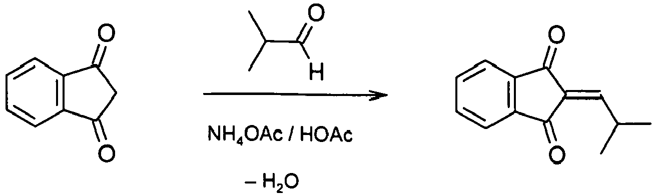

1,3-Indandione (1.46 g, 10 mmol), isobutyryl aldehyde (1,08 g, 15 mmol), ammonium acetate (0,13 g, 1,7 mmol) and acetic acid (0,3 ml) were dissolved in 20 ml of chloroform and refluxed for 150 min with a dean-stark trap. The dark red solution is washed with distilled water until the water phase is neutral, then the organic phase is dried with MgSO₄. After evaporation of the chloroform, the residue is crystallized from methanol/water; m.p. 56-58 °C. Yield: 1,07 g (53%) of 2-isobutylidene-indandione-1,3.

### Synthesis Example 2 - Synthesis of 2-Benzylidene-indandione-1,3

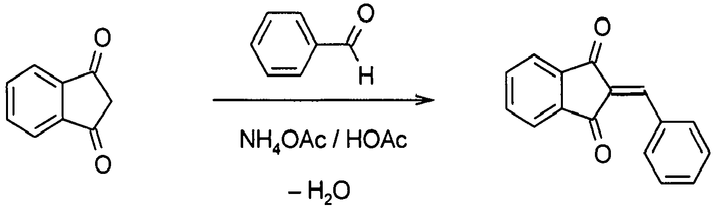

1,3-Indandione (1.46 g, 10 mmol), benzaldehyde (1,59 g, 15 mmol), ammonium acetate (0,13 g, 1,7 mmol) and acetic acid (0,3 ml) were dissolved in 20 ml of chloroform and refluxed for 150 min with a dean-stark trap. The dark red solution is washed with distilled water until the water phase is neutral, then the organic phase is dried with MgSO₄. After evaporation of the chloroform, the residue is crystallized from methanol/water; m.p. 152-56 °C. Yield: 1,05 g (45%) of 2-benzylidene-indandione-1,3.

### Example 1 - Synthesis of Compound (Ia)

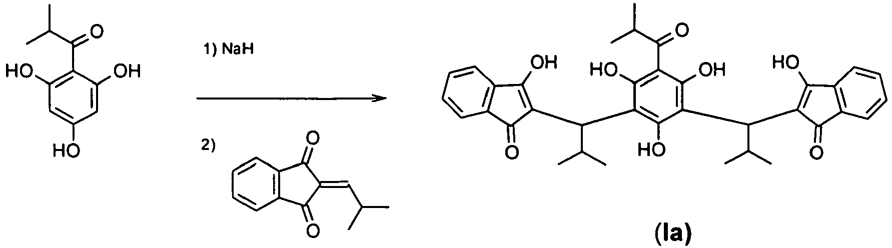

Sodium hydride dispersion in mineral oil (80 mg of a 60% dispersion corresponds to 48 mg NaH, 2 mmol) is washed two times with 2 ml of dry THF under nitrogen to remove the mineral oil. Then the NaH is suspended in 5 ml of dry THF and a solution of isobutyryl phloroglucinole (196 mg, 1 mmol) in 15 ml of dry THF is added. The reaction mixture is stirred for 10 min. until hydrogen evolution ceases. Then, a solution of 2- isobutylidene-indandione-1,3 (800 mg, 4 mmol) in 5 ml of dry THF is added and the reaction mixture is stirred until the starting phloroglucinol is consumed (reaction monitoring by tlc; reaction time ca. 1 h). Evaporation of the solvent results in a crude product, which is purified by flash-chromatography (petroleum ether/acetone 3:2 (v/v); R_{f} = 0.27). Product (**Ia**) is obtained as a red solid with a melting range of 100-140 °C; yield: 507 mg (0.85 mmol; 85%). The melting range is due to the fact that the product is a mixture of the racemate and the meso-form of (**Ia**).

### Example 2 - Synthesis of Compound (Ib)

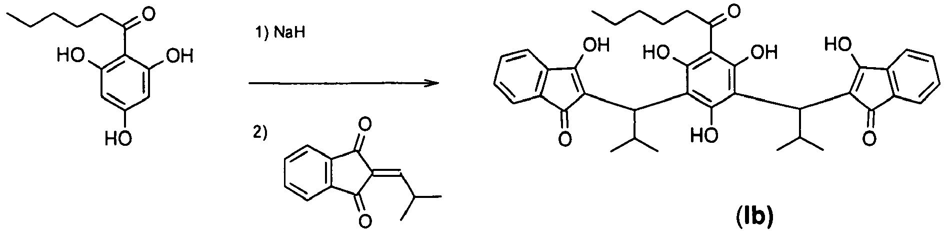

Sodium hydride dispersion in mineral oil (80 mg of a 60% dispersion corresponds to 48 mg NaH, 2 mmol) is washed two times with 2 ml of dry THF under nitrogen to remove the mineral oil. Then the NaH is suspended in 5 ml of dry THF and a solution of hexanoyl phloroglucinole (224 mg, 1 mmol) in 15 ml of dry THF is added. The reaction mixture is stirred for 10 min. until hydrogen evolution ceases. Then, a solution of 2- isobutylidene-indandione-1,3 (800 mg, 4 mmol) in 5 ml of dry THF is added and the reaction mixture is stirred until the starting phloroglucinol is consumed (reaction monitoring by tlc; reaction time ca. 1 h). Evaporation of the solvent results in a crude product, which is purified by flash-chromatography (petroleum ether/acetone 3:2 (v/v); R_{f} = 0.27). Product **(Ib)** is obtained as a red solid with a melting range of 100-145 °C; yield: 487 mg (0.78 mmol; 78%). The melting range is due to the fact that the product is a mixture of the racemate and the meso-form of **(Ib).**

### Example 3 - Synthesis of Compound (Ic)

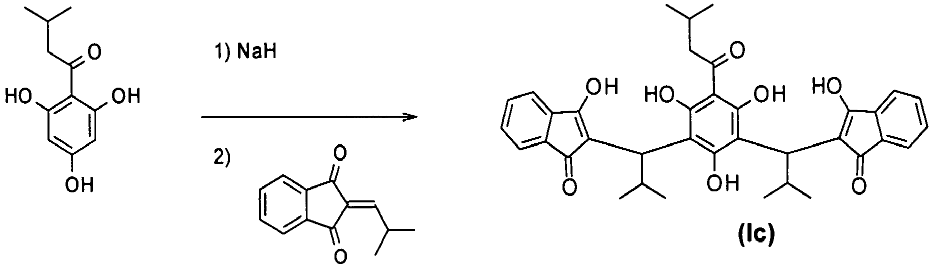

Sodium hydride dispersion in mineral oil (80 mg of a 60% dispersion corresponds to 48 mg NaH, 2 mmol) is washed two times with 2 ml of dry THF under nitrogen to remove the mineral oil. Then the NaH is suspended in 5 ml of dry THF and a solution of isovaleroyl phloroglucinole (210 mg, 1 mmol) in 15 ml of dry THF is added. The reaction mixture is stirred for 10 min. until hydrogen evolution ceases. Then, a solution of 2- isobutylidene-indandione-1,3 (800 mg, 4 mmol) in 5 ml of dry THF is added and the reaction mixture is stirred until the starting phloroglucinol is consumed (reaction monitoring by tlc; reaction time ca. 1 h). Evaporation of the solvent results in a crude product, which is purified by flash-chromatography (petroleum ether/acetone 3:2 (v/v); R_{f} = 0.25). Product **(Ic)** is obtained as a red solid with a melting range of 100-145 °C; yield: 470 mg (0.77 mmol; 77%). The melting range is due to the fact that the product is a mixture of the racemate and the meso-form of **(Ic).**

### Example 4 - Synthesis of Compound (Id)

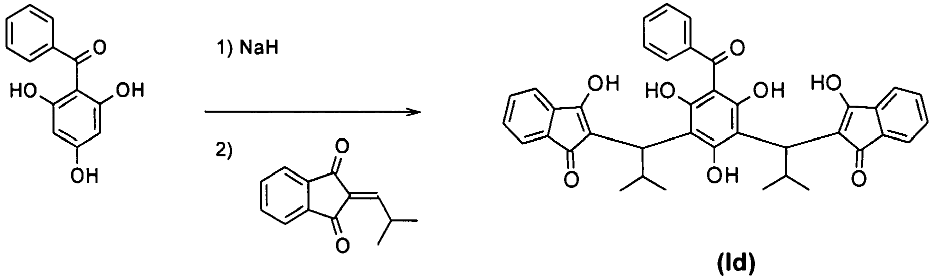

Sodium hydride dispersion in mineral oil (80 mg of a 60% dispersion corresponds to 48 mg NaH, 2 mmol) is washed two times with 2 ml of dry THF under nitrogen to remove the mineral oil. Then the NaH is suspended in 5 ml of dry THF and a solution of benzoyl phloroglucinole (230 mg, 1 mmol) in 15 ml of dry THF is added. The reaction mixture is stirred for 10 min. until hydrogen evolution ceases. Then, a solution of 2- isobutylidene-indandione-1,3 (800 mg, 4 mmol) in 5 ml of dry THF is added and the reaction mixture is stirred until the starting phloroglucinol is consumed (reaction monitoring by tlc; reaction time ca. 1 h). Evaporation of the solvent results in a crude product, which is purified by flash-chromatography (petroleum ether/acetone 3:2 (v/v); R_{f} = 0.15). Product **(Id)** is obtained as a red solid with a melting range of 100-150 °C; yield: 486 mg (0.77 mmol; 77%). The melting range is due to the fact that the product is a mixture of the racemate and the meso-form of **(Id).**

### Example 5 - Synthesis of Compound (Ie)

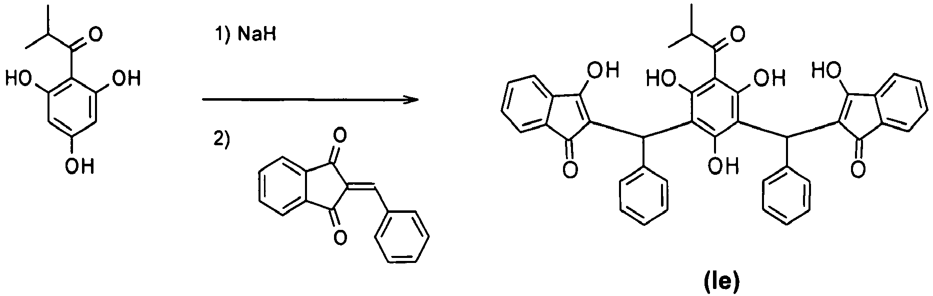

Sodium hydride dispersion in mineral oil (80 mg of a 60% dispersion corresponds to 48 mg NaH, 2 mmol) is washed two times with 2 ml of dry THF under nitrogen to remove the mineral oil. Then the NaH is suspended in 5 ml of dry THF and a solution of isobutyryl phloroglucinole (196 mg, 1 mmol) in 15 ml of dry THF is added. The reaction mixture is stirred for 10 min. until hydrogen evolution ceases. Then, a solution of 2-benzylidene-indandione-1,3 (700 mg, 3 mmol) in 5 ml of dry THF is added and the reaction mixture is stirred until the starting phloroglucinol is consumed (reaction monitoring by tlc; reaction time ca. 1 h). Evaporation of the solvent results in a crude product, which is purified by flash-chromatography (petroleum ether/acetone 1:1 (v/v); R_{f} = 0.19). Product **(Ie)** is obtained as a orange solid with a melting range of 120-165 °C; yield: 592 mg (0.89 mmol; 89%). The melting range is due to the fact that the product is a mixture of the racemate and the meso-form of **(Ie).**

### Example 6 - Synthesis of Compound (1f)

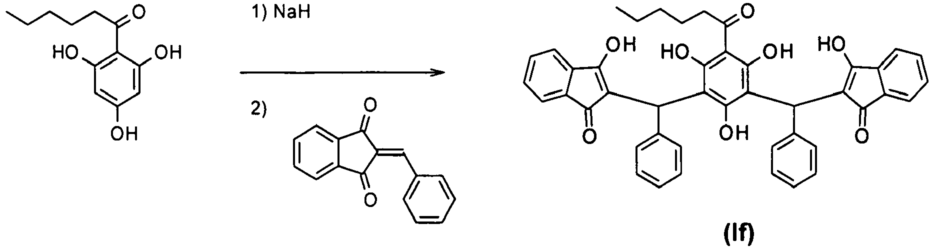

Sodium hydride dispersion in mineral oil (80 mg of a 60% dispersion corresponds to 48 mg NaH, 2 mmol) is washed two times with 2 ml of dry THF under nitrogen to remove the mineral oil. Then the NaH is suspended in 5 ml of dry THF and a solution of hexanoyl phloroglucinole (224 mg, 1 mmol) in 15 ml of dry THF is added. The reaction mixture is stirred for 10 min. until hydrogen evolution ceases. Then, a solution of 2-benzylidene-indandione-1,3 (700 mg, 3 mmol) in 5 ml of dry THF is added and the reaction mixture is stirred until the starting phloroglucinol is consumed (reaction monitoring by tlc; reaction time ca. 1 h). Evaporation of the solvent results in a crude product, which is purified by flash-chromatography (petroleum ether/acetone 1:1 (v/v); R_{f} = 0.15). Product **(If)** is obtained as an orange solid with a melting range of 80-125 °C; yield: 623 mg (0.90 mmol; 90%). The melting range is due to the fact that the product is a mixture of the racemate and the meso-form of **(If).**

### Example 7 - Synthesis of Compound (Ig)

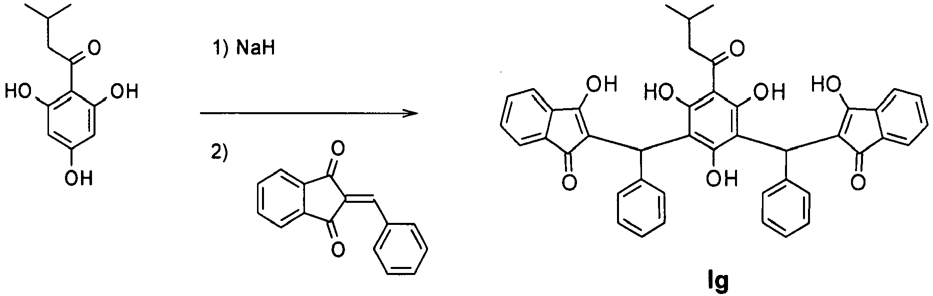

Sodium hydride dispersion in mineral oil (80 mg of a 60% dispersion corresponds to 48 mg NaH, 2 mmol) is washed two times with 2 ml of dry THF under nitrogen to remove the mineral oil. Then the NaH is suspended in 5 ml of dry THF and a solution of isovaleroyl phloroglucinole (210 mg, 1 mmol) in 15 ml of dry THF is added. The reaction mixture is stirred for 10 min. until hydrogen evolution ceases. Then, a solution of 2-benzylidene-indandione-1,3 (700 mg, 3 mmol) in 5 ml of dry THF is added and the reaction mixture is stirred until the starting phloroglucinol is consumed (reaction monitoring by tlc; reaction time ca. 1 h). Evaporation of the solvent results in a crude product, which is purified by flash-chromatography (petroleum ether/acetone 1:1 (v/v); R_{f} = 0.24). Product **(Ig)** is obtained as an orange solid with a melting range of 90-130 °C; yield: 638 mg (0.94 mmol; 94%). The melting range is due to the fact, that the product is a mixture of the racemate and the meso-form of **(Ig).**

### Example 8 - Synthesis of Compound (1 h)

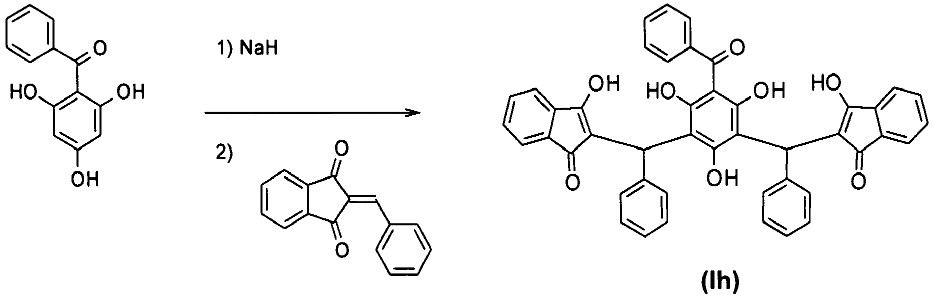

Sodium hydride dispersion in mineral oil (80 mg of a 60% dispersion corresponds to 48 mg NaH, 2 mmol) is washed two times with 2 ml of dry THF under nitrogen to remove the mineral oil. Then the NaH is suspended in 5 ml of dry THF and a solution of benzoyl phloroglucinole (230 mg, 1 mmol) in 15 ml of dry THF is added. The reaction mixture is stirred for 10 min. until hydrogen evolution ceases. Then, a solution of 2-benzylidene-indandione-1,3 (700 mg, 3 mmol) in 5 ml of dry THF is added and the reaction mixture is stirred until the starting phloroglucinol is consumed (reaction monitoring by tlc; reaction time ca. 1 h). Evaporation of the solvent results in a crude product, which is purified by flash-chromatography (petroleum ether/acetone 1:1 (v/v); R_{f} = 0.24). Product **(Ih)** is obtained as an orange solid with a melting range of 90-130 °C; yield: 643 mg (0.92 mmol; 92%). The melting range is due to the fact that the product is a mixture of the racemate and the meso-form of **(Ih).**

### Example 9 - Determination of PGE2 synthase activity in microsomes of A549 cells

(A. Koeberle, F. Pollastro, H. Northoff and O. Werz, Br. J. Pharmacol. 2009, 156, 952-961.)

Microsomal membranes were diluted in potassium phosphate buffer (0.1 mol·1⁻¹, pH 7.4) containing 2.5 mmol·l⁻¹ glutathione.

Test compounds or vehicle were added, and after 15 min at 4°C the reaction (100 ml total volume) was initiated by addition of PGH2 (20 mmol·l⁻¹, final concentration). After 1 min at 4°C, the reaction was terminated by using stop solution (100 ml; 40 mmol·l⁻¹ FeCl2, 80 mmol·l⁻¹ citric acid and 10 mmol·l⁻¹ of 11b-PGE2). PGE2 was separated by solid phase extraction on reversed phase (RP)-C18 material by using acetonitrile (200 ml) as eluent, and analysed by RP-HPLC (30% acetonitrile in water + 0.007% TFA (v,v), Nova-Pak® C18 column, 5 x 100 mm, 4 mm particle size, flow rate 1 m-·min⁻¹) with UV detection at 195 nm. 11b-PGE2 was used as internal standard to quantify PGE2 product formation by integration of the area under the peaks.

Results for compounds of the present invention are shown in Table 1.

### Example 10 - Determination of 5- and 15-Lipoxygenase Products in PMNLs

(C. Feißt, L. Franke, G. Appendino and O. Werz, J. Pharm. Exp. Ther. 2005, 315, 3899-396.)

To assay 5- and 15-lipoxygease (LO) product formation in intact cells, 7.5 x 10⁶ freshly isolated PMNLs were finally resuspended in 1 ml of PGC buffer. After preincubation with the test compounds for 10 min at 37°C, the reaction was started by the addition of 1 µM ionomycin plus 20 µM AA. After 10 min at 37°C, the reaction was stopped with 1 ml of methanol and 30 µl of 1 N HCl, and 200 ng of prostaglandin B1 and 500 µl of PBS were added. Formed AA metabolites were extracted and analyzed by HPLC. To determine product formation of purified 5-LO enzyme, 5-LO protein (0.1 µg in 10 µl) was added to 990 µl of PBS containing 1 mM EDTA and 1 mM ATP on ice and the indicated compounds were added. After 5 to 10 min on ice, the samples were preincubated for 30 s at 37°C and CaCl₂ and AA (2 mM and 10 µM, respectively) were added to start the 5-LO reaction. After 10 min, the incubation was terminated and 5-LO product formation was determined as described for the intact cells. 15-LO product formation is expressed as nanograms of 15(S)-hydro(peroxy-5,8,11-*cis*-13-*trans*-eicosatetraenoic acid per 10⁶ cells. 5-LO product formation is expressed as nanograms of 5-LO products per 106 cells, which include LTB4 and its all-*trans*-isomers, 5(*S*),12(*S*)-di-hydroxy-6,10-*trans*-8,14-cis-eicosatetraenoic acid and 5(*S*)-hydro(pero)xy-6-*trans*-8,11,14-cis-eicosatetraenoic acid. Cysteinyl LTs (LTC4, D4, and E4) were not detected, and oxidation products of LTB4 were not determined.

Results for compounds of the present invention are shown in Table 1.

### Example 11 - Determination of cell viability by MTT assay

(I. Tretiakova, D. Blaesius, L. Maxia, S. Wesselborg, K. Schulze-Osthoff, J CinatlJr., M. Michaelis, O. Werz, Apoptosis 2008, 13, 119-131.)

Cell viability was assessed using the MTT assay. Cell lines were incubated at 37°C and 5% CO2 atmosphere with the indicated additives for 5 days. PBMC were treated for 40 h and in some experiments, MM6 cells were only treated for 24 h. DMSO was used as solvent never exceeding 0.5% (vol/vol). Then, MTT reagent was added for 4 h. Thereafter, 100 II SDS solution (20% SDS in a 1:1 (vol by vol) dimethylformamide/ water solution) was added for 4 h. Plates were read on a multiwell scanning spectrophotometer (Victor3 plate reader, PerkinElmer, Rodgau-Juegesheim, Germany) at a wavelength of 620 nm and a reference wavelength of 690 nm. Induction of cell death was determined as the relative reduction of the optical density.

Results for compounds of the present invention are shown in Table 1.

The entire disclosures of all references cited herein, such as patent documents, books and journal articles, are hereby incorporated into this specification by reference.

**Table 1**

| Inhibition values for mPGES-1 and for 5-LO by the 1,3-indandione derivatives as well as apoptose induction | | | | |
|---|---|---|---|---|
| Structure | mPGES-1 (IC50 in µmol l⁻¹) | 5-LO cell-free assay (IC50 in µmol l⁻¹) | 5-LO cell-based assay (IC50 in µmol l⁻¹) | Apoptose induction (EC₅₀ in µmol l⁻¹) |
| 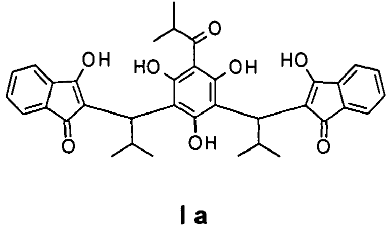 | 0.30 | 2.80 | 6.70 | > 30 |
| 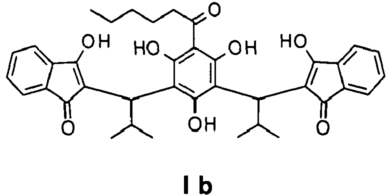 | 0.08 | 1.74 | 1.46 | > 30 |
| 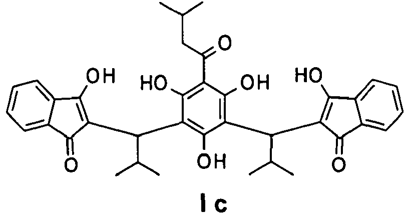 | 0.18 | 0.30 | 1.04 | > 30 |
| 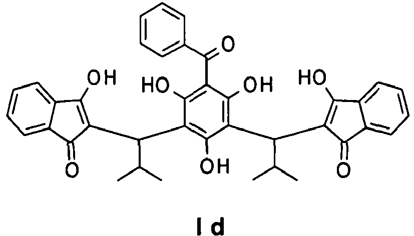 | 0.68 | 6.86 | 3.23 | > 30 |
| 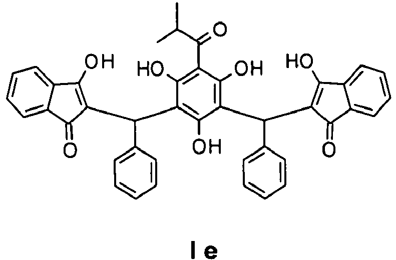 | 0.55 | 0.73 | 0.62 | > 30 |
| 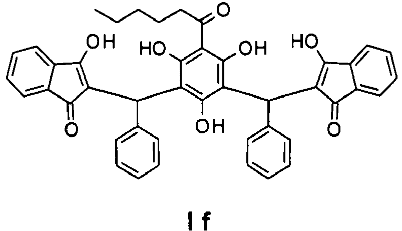 | 0.19 | 0.46 | 0.69 | > 30 |
| 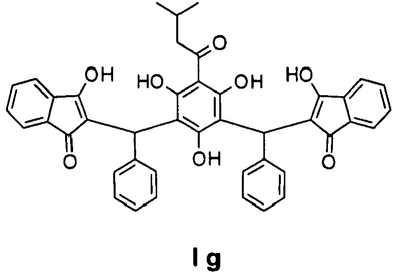 | 0.39 | 1.1 | 2.09 | > 30 |
| 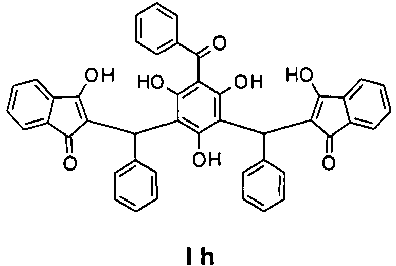 | 0.33 | 2.99 | 0.84 | > 30 |

## Claims

**1.** A Myrtucommulone analogue of general formula (I) 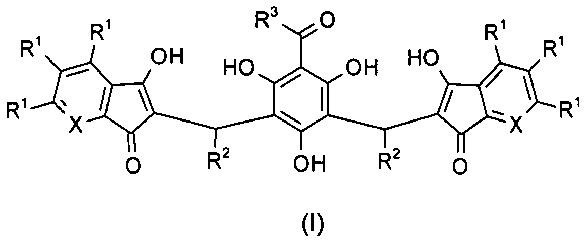 wherein
X is N or CR¹;
each R¹ is independently of each other selected from H and a C₁₋₁₂ linear, branched or cyclic alkyl, allyl or aryl-C₁₋₄ alkyl group wherein the aryl may be substituted by 1 to 5 substituents independently selected from C₁₋₄ alkyl, C₁₋₄ alkoxy, nitro, halogen (F, Cl, Br und I), cyano, -C(O)-R' and -COOR', wherein R' is C₁₋₄ alkyl, with the proviso that the two indanolone rings are substituted identically;
each R² is the same substituent and is selected from H and a C₁₋₁₂ linear, branched or cyclic alkyl, allyl, aryl, or aromatic heterocyclic group comprising an aromatic 6-membered ring including one or two nitrogen atoms or an aromatic 5-membered ring including one or two heteroatoms selected from nitrogen and not more than one oxygen atom and not more than one sulfur atom per ring, wherein aryl or the aromatic heterocyclic group may be substituted by one or more substituents independently selected from C₁₋₄ alkyl, C₁₋₄ alkoxy, nitro, halogen (F, Cl, Br und I), cyano, dimethylamino, hydroxyl, -C(O)-R' and -COOR', wherein R' is C₁₋₄ alkyl or H;
R³ is a C₁₋₁₂ linear, branched or cyclic alkyl group, which may be substituted with CO₂H or SO₃H; or an allyl, aryl or aromatic heterocyclic group comprising an aromatic 6-membered ring including one or two nitrogen atoms or an aromatic 5-membered ring including one or two heteroatoms selected from nitrogen and not more than one oxygen atom and not more than one sulfur atom per ring, wherein aryl or the aromatic heterocyclic group may be substituted by one or more substituents independently selected from C₁₋₄ alkyl, C₁₋₄ alkoxy, nitro, halogen (F, Cl, Br und I), cyano, dimethylamino, hydroxyl, -C(O)-R' and -COOR', wherein R' is C₁₋₄ alkyl or H.

**2.** A Myrtucommulone analogue according to claim 1, wherein each R¹ is independently selected from the group consisting of H and methyl.

**3.** A Myrtucommulone analogue according to claim 1 or 2, wherein each R² is isopropyl or phenyl.

**4.** A Myrtucommulone analogue according to any of claims 1 to 3, wherein R⁴ is selected from the group consisting of isopropyl, butyl, pentyl, hexyl and phenyl.

**6.** A Myrtucommulone analogue, which is selected from
2,2'-(1,1'-(2,4,6-Trihydroxy-5-isobutyryl-1,3-phenylene)bis(2-methylpropane-1,1-diyl))bis(3-hydroxy-1H-inden-1-one) 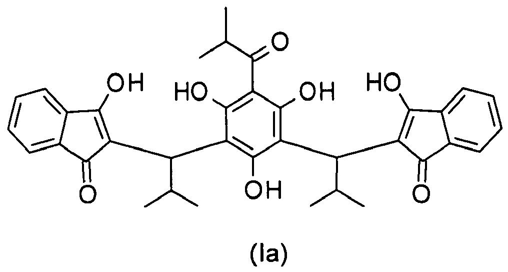 2,2'-(1,1'-(5-Hexanoyl-2,4,6-trihydroxy-1,3-phenylene)bis(2-methylpropane-1,1-diyl))bis(3-hydroxy-1H-inden-1-one) 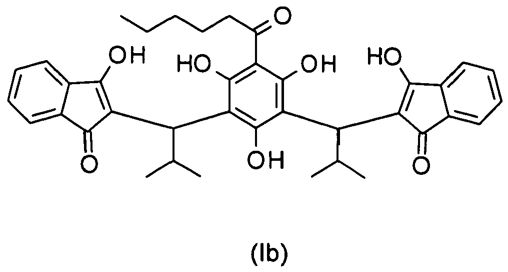 2,2'-(1,1'-(2,4,6-Trihydroxy-5-(3-methylbutanoyl)-1,3-phenylene)bis(2-methylpropane-1,1-diyl))bis(3-hydroxy-1H-inden-1-one) 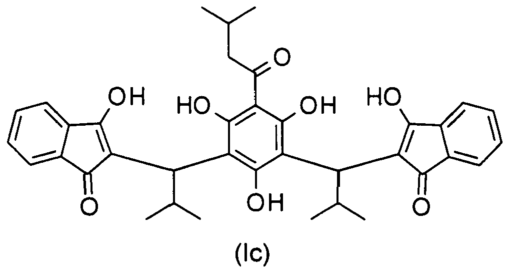 2,2'-(1,1'-(5-Benzoyl-2,4,6-trihydroxy-1,3-phenylene)bis(2-methylpropane-1,1-diyl))bis(3-hydroxy-1H-inden-1-one) 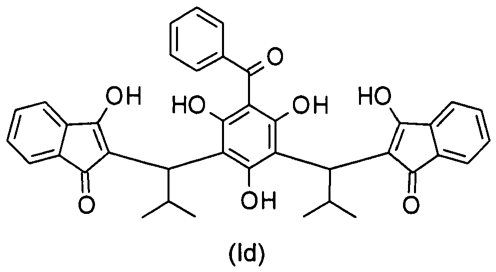 2,2'-(2,4,6-Trihydroxy-5-isobutyryl-1,3-phenylene)bis(phenylmethylene)-bis(3-hydroxy-1H-inden-1-one) 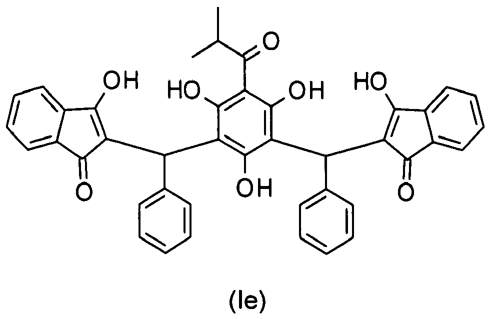 2,2'-(2,4,6-Trihydroxy-5-(3-methylbutanoyl)-1,3-phenylene)bis-(phenylmethylene)bis(3-hydroxy-1H-inden-1-one) 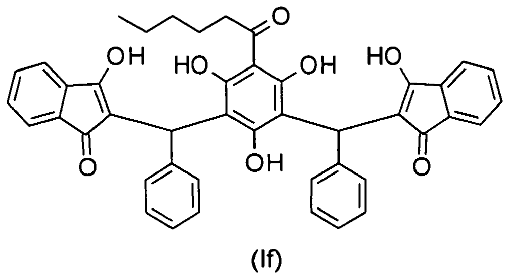 2,2'-(5-Hexanoyl-2,4,6-trihydroxy-1,3-phenylene)bis(phenylmethylene)bis(3-hydroxy-1H-inden-1-one) 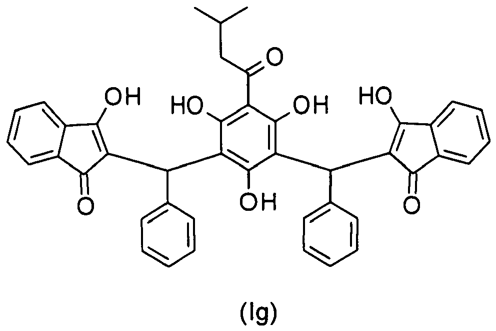 and
2,2'-(5-Benzoyl-2,4,6-trihydroxy-1,3-phenylene)bis(phenylmethylene)bis(3-hydroxy-1H-inden-1-one)

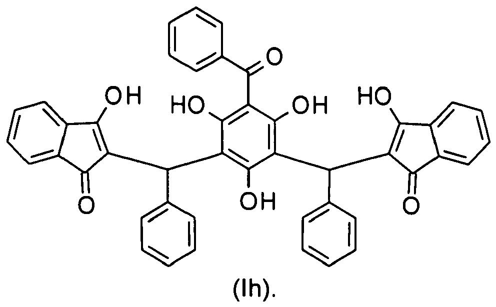

**7.** A method of preparing the Myrtucommulone analogue of claim 1 comprising:
(a) dissolving a compound of formula (IV) 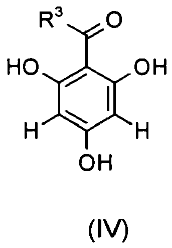 wherein R³ is as defined in claim 1,
in an aprotic solvent and reacting it with about 1 to about 3 equivalents of a strong base to give a solution or a suspension of a salt of the compound of formula (IV);
(b) dissolving a compound of formula (III) 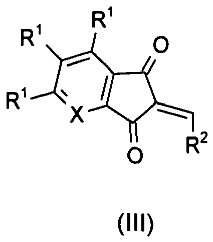 wherein X, R¹ and R² are as defined in claim 1,
in an aprotic solvent; and
(c) combining the reaction product of (a) with the solution of (b) to obtain
a compound of formula (I) 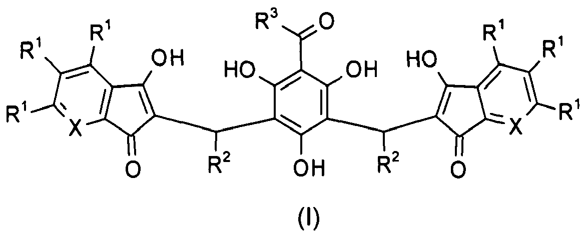 wherein X, R¹, R² and R³ are as defined above.

**8.** A method according to claim 7, wherein each R¹ is independently selected from H or methyl.

**9.** A method according to claim 7 or 8, wherein each R² is isopropyl or phenyl.

**10.** A method according to any of claims 7 to 10, wherein R⁴ is selected from the group consisting of isopropyl, butyl, pentyl, hexyl and phenyl.
